Europäisches Patentamt

European Patent Office

Office européen des brevets

(11) Publication number: **0 595 006 A1**

# EUROPEAN PATENT APPLICATION

(21) Application number: **93114765.6**

(22) Date of filing: **14.09.93**

(51) Int. Cl.5: **A61K  31/68**, //(A61K31/68, 31:505,31:44)

(30) Priority: **14.09.92 ZA 926989**

(43) Date of publication of application:
**04.05.94 Bulletin  94/18**

(84) Designated Contracting States:
**AT BE CH DE DK ES FR GB GR IT LI LU NL SE**

(71) Applicant: **VESTA MEDICINES (PROPRIETARY) LIMITED**
**Holpro House**
**1 Snell Street**
**Micor, Johannesburg 2092(ZA)**

(72) Inventor: **Serfontein, Willem Jacob**
**47 Selikats Village,**
**Selikats Causeway**
**Faerie Glen, Pretoria 0043(ZA)**

(74) Representative: **VOSSIUS & PARTNER**
**Postfach 86 07 67**
**D-81634 München (DE)**

(54) **Pharmaceutical and dietary compositions for the treatment and prophylaxis of metabolic disturbances in infants containing vitamin B6, folic acid and vitamin B12.**

(57) Pharmaceutical respectively dietary preparations are disclosed for the treatment or prophylaxis of elevated homocysteine and/or methionine levels in the blood of human infants and pathological disturbances connected therewith, said preparation comprising in combination:-

a) vitamin B 6 as such or in the form of a pharmaceutically acceptable acid salt, at least in part in the form of pyridoxal (PL) or a compound which in vivo readily releases PL without the intervention of oxidase enzyme or oxygen.

b) folate or a precursor of folate which releases folate in vivo, and

c) vitamin B12, with or without intrinsic factor, in the following ratios:-

a) : b) from 1:25 to 10 000 : 1

b) : c) from 1:1 to 50 000 : 1

The preparations are to be incorporated in infant bottle feed mixes.

EP 0 595 006 A1

The present invention relates to compositions for the treatment and prophylaxis of metabolic disturbances in infants.

The relationship between even moderately elevated blood homocysteine levels homocysteineaemia and various pathological conditions, in particular vascular and neurological diseases has been firmly established in adults. (Ueland et al, Scan. J. Clin. Lab. Invest. 1988, 48:215)

Hitherto, this problem was considered to primarily affect adults and possibly adolescents, since it was primarily believed to be connected with long term dietary factors, possibly enhanced by genetic factors.

It therefore came as a great surprise, when the present applicants conducted homocysteine measurements in the serum of new born and premature infants (something which had never before been reported) and discovered an amazing frequency of homocysteine abnormalities in such infants in addition to a number of important additional findings.

It was found that average homocysteine levels in healthy infants (6 - 7 $\mu$ mole/$l$ are much lower than in adults (for whom up to 16,3 $\mu$ mole/$l$ is normal). However, surprisingly a relatively large proportion of infants were found to have relatively elevated homocysteine levels. Based on experiments conducted by the applicant on adults and various blood measurements carried out on the infants, it was possible to reach conclusions as to causes and potential consequences, and the present invention proposes appropriate countermeasures.

It was found that in ideal circumstances healthy infants, from healthy mothers and wholly breast fed are protected naturally against elevated homocysteine levels, a fact which indirectly confirms the danger residing in homocysteine. In healthy new-born infants plasma levels of pyridoxal, vitamin B12 and folate were found to be considerably higher than in adults. Applicant has established that these high levels provide a natural protective mechanism against accumulation of toxic homocysteine levels.

It was possible to correlate deficiencies in respect of one or more of the aforegoing with elevated homocysteine levels in the respective infants and detect connections with genetic factors, with pre-natal and post-natal maternal nutritional status (the latter being relevant to breast fed infants). However, most alarming of all was the discovery of the extent to which these elevated homocysteine levels can be ascribed to bottle feeding with non-human milk and milk formulae based on cow's milk. A careful assessment of certain differences between human and non-human milk has revealed plausible causes for the prevalence of homocysteineaemia amongst infants. In particular, cow's milk contains 4 times more methionine than mother's milk yet is lacking proportionally in factors which would prevent or counteract the transformation of this excessive amount of methionine into homocysteine.

Fraying and splitting of the vascular internal elastic membranes has been observed as a clinical symptom of elevated homocysteine levels in adults. This same symptom was described by Jaffe in 176 babies who had died of different causes during their first 3 months of life. Sudden infant death syndrome has been found to be particularly common amongst bottle-fed babies.

The vascular damage caused by homocysteine is well established. Research has shown that this kind of damage is the primary cause of and precondition for subsequent vascular damage by cholesterol. Research has further revealed that cholesterol-rich lipoproteins only becomes dangerous once it has been oxidised to oxycholesterol, either in the diet or in vivo. This is brought about by the intervention of free radicals. In this context it is particularly alarming that homocysteine itself is a known stimulant for free radical induced oxidation of lipproteins, including that of cholesterol, this besides other harmful effects of free radicals in vivo. Moreover, in a manner to be explained further below, thermally processed bottle feed products are themselves rich in oxycholesterol but are deficient in counteracting substances present in human milk.

Even if the adverse factors described above, in particular elevated homocysteine result in no immediately apparent symptoms, they may predispose to atherosclrosis in later life.

The damage caused by homocysteine in infants is obviously not limited to the vascular system but may include many other tissues and systems including the central nervous system, which is particularly vulnerable at this stage.

The invention is inter alia based on the discovery of the above described hitherto unknown problem and seeks solutions to this and/or related problems.

The present invention provides a new use of the substances hereinafter set out for the manufacture of a new pharmaceutical or dietary preparation for the treatment or prophylaxis of elevated homocysteine and/or methionine levels in the blood of human infants and pathological disturbances connected therewith, said preparation comprising in combination:-

a) vitamin B 6 as such or in the form of a pharmaceutically acceptable acid salt, at least in part in the form of pyridoxal (PL) or a compound which in vivo readily releases PL without the intervention of oxidase enzyme or oxygen.

b) folate or a precursor of folate which releases folate in vivo, and

c) vitamin B12, with or without intrinsic factor, in the following ratios:-

a):b) from 1:25 to 10000:1

preferably from 2:5 to 150:1

more preferably from 13:1 to 100:1

e.g. 15:2, and

b):c) from 1:1 to 50000:1

preferably from 2:1 to 4000:1

more preferably from 10:1 to 1000:1

e.g. 400:11

Masons et al, Brit.Med.J. (1973), 1, 389-390, report successful treatments of acquired sideroplastic anaemia with intramuscular pyridoxal-5-phosphate (PLP) in cases which did not respond to PN. It was not realised that, as taught by the present invention, the administration of unphosphorylated PL, even by the oral route, would have been preferable and would have eliminated a further protential bottleneck in the metabolic pathway.

Pharmaceutical preparations containing the aforesaid active ingredients have been described, albeit for totally different purposes and mostly in ratios differing from the aforesaid ratios or at least from the preferred or more preferred ratios. In GB-PS 1201 014 (Examples 6 and 7) the ratio of a):b) = 3:1 and that of b):c) = 1000:5. No indication is disclosed for these dosages which, however, are clearly not intended for paediatric use. GB-PS 2254 556, published after the priority date of the present disclosure also discloses compositions, only some of which contain in combination folic acid, vitamin B12 and vitamin B6. No distinction is drawn between pyridoxine, pyridoxal and pyridoxamine. These compositions are intended for adolescent girls. GB-PS 149 3993 discloses compositions for treating obesity. Pyridoxal is not disclosed.

GB-PS 2197 587 describes a "blood conditioning tonic" for race horses. Pyridoxal is not disclosed. GB-PS 1431 841 discloses preparations for cataract treatment. The ratios are different from those according to the invention and pyridoxal is not disclosed. GB 101 3939 discloses compositions for paediatric purposes in ratios which overlap the broadest ratio according to the invention, but the important feature that the vitamin B6 must be in the form of pyridoxal or suitable precursor thereof is not disclosed. This also applies to EP-0144051, EP 0121 036 or PCT WO 83/00085.

Not one of the aforesaid references discloses such combinations for the treatment or prophylaxis of elevated homocysteine levels in infants, or the crucial role of pyridoxal in that context.

The preparation in accordance with the invention can be galenically prepared for parenteral administration, e.g. by infusion. However, the preferred dosage forms are for oral use.

It is very important for optimum effects for the daily dosage to be spread as evenly as possible over a 24 hour period. This is best achieved in the case of oral administration, if the preparation is administered regularly at feeding times, e.g. as part of a bottle-feeding programme. For that purpose the preparation may take the form of paediatric drops or of dosage units such as soluble tablets, rapidly disintegrating tablets- or powders or granulates prepacked into sachets. Powders or granulates may also be provided with a measuring spoon or scoop for determining the desired dose.

The composition may also be incorporated in a bottle-feed powdered milk formula.

The present invention has certain features in common with the invention forming the subject of our co-pending application of even dated entitled "Pharmaceutical Preparations for Lowering Homocysteine Levels". Here pharmaceutical preparations for lowering blood and tissue levels of homocysteine are disclosed, comprising:

a) vitamin B6;

b) folate or a suitable active metabolite of folate or a substance which releases folate in vivo;

c) vitamin B12, with or without intrinsic factor

and optionally antoxidants, choline and/or betaine. a) and b) are provided in slow release form (2-8 hours) and c) is to be released immediately (within 20 minutes).

Relevant disclosures thereof are to be considered a part of the present disclosure. Also, as regards suitable precursors for pyridoxal, reference is made to the teachings of European patent application 90 100 834.2 and corresponding applications in other countries which by reference thereto are to be considered as part of the present disclosure.

It has surprisingly been found that PL serum levels in healthy new-born babies are very much elevated, thus underlining the importance of PL in the metabolism of infants. This is in line with the fact that human milk in contrast to cow's milk or goat's milk has a high PL content and little or no PN content. New-born infants, particularly when premature, are enzymatically poorly equipped to convert pyridoxine (PN), the conventional source of vitamin B6 in non-human milk and conventional infant milk formulae, into the required PL. Whilst this has already been disclosed in the aforegoing European patent application 90 100 834.2, the present invention provides a new and even more pressing reason for the need in infants to supplement plasma PL. Plasma PL is the only form of vitamin B6 capable of entering most cells in humans through the cellular membranes, in order to be immediately converted into intracellular pyridoxal phosphate (PLP), the only active form of vitamin B6 in humans. Also the PL form of vitamin B6 is the form needed as a co-factor for the enzyme cystathione synthase, which serves to convert homocysteine into cysteine by the transsulfuration pathway. This reaction reduces the methionine pool in the body and is accordingly important to limit the re-formation of homocysteine. Infants deficient in plasma PL lack the ability to degrade homocysteine, which may then lead to homocysteineaemia.

Prior art attempts at counteracting PL deficiency in infants by high dosage rates of PN, particularly when in bolus form, can be dangerous and result in toxicity effects similar to those of a depressed vitamin B6 status. As more fully explained in the aforesaid European patent application, this is due to competitive inhibition of the PN-PLP (intracellular) pathway by excessive supplies of PN.

This is the reason why in accordance with the present invention it is particularly important to supplement plasma PL by the administration of vitamin B6 at least in part in the form of PL or an appropriate precursor of PL which does not depend on the intervention of oxidase or oxygen.

It is also preferred to employ such PL or precursor in a nonphosphorylated form in order to bypass dephosphorylation reactions induced by phosphatase enzymes.

Nevertheless, in order to stimulate the development of the oxidase enzyme system in the infant it is preferred to include modest amounts of PN or PN precursor in the preparation in addition to PL.

The ratio of PN:PL may be in the range 10:1 to 1:10, preferably 4:1 to 1:6 more preferably 2:1 to 1:3 e.g. 1:2.

Because the oxidase enzyme system is very sensitive to riboflavin deficiencies it is advisable to incorporate in the preparation riboflavin, e.g. in an amount of from 0,5 to 5 times, e.g. twice the amount of PN.

The daily dosages of the aforesaid ingredients per infant are:

| Component | Range | Preferred | More Preferred | Example |
|-----------|-------|-----------|----------------|---------|
|           | mg    | mg        | mg             | mg      |
| PL        | 0,01-5 | 0,05-1,5 | 0,08-0,5 | 0,20 |
| PN        | 0,01-5 | 0,03-0,5 | 0,05-1,5 | 0,1 |
| Riboflavin | 0,01-5 | 0,05-3,0 | 0,1-2 | 0,2 |
| Folate    | 0,001-0,5 | 0,01-0,2 | 0,02-0,1 | 0,04 |
| Vit B12   | 0,01-10mcg | 0,05-5mcg | 0,1-2mcg | 0,55 mcg |

Homocysteine, besides the various toxic effects already mentioned, also stimulates free radical induced oxidation, including that of cholesterol. This adds to the vascular damage directly induced by homocysteine, because once that first damage has been done, the blood vessels so damaged are in a condition in which they are vulnerable to the atherosclerotic action of cholesterol, and that action is performed by cholesterol mostly in its oxidised form.

This adverse effect is aggravated in infants by other factors, particularly in the case of bottle-fed babies. It has now been found that the thermal processes to which substitutes for human milk are subjected for bottle feeding purposes - boiling, spray drying - likewise result in the formation of dangerous oxy-cholesterol from the originally unoxidised lipids present in natural fresh milk.

In addition, applicant has surprisingly found that cow's milk besides containing four times more methionine than human milk, contains 6 times less selenium, 2-3 times less vitamin E, 4-6 times less vitamin C, 3 times less vitamin A and 2-3 times less nicotinic acid. In the context of the present invention it is pointed out that vitamin C and E are important anti-oxidants by virtue of their ability to react directly with free radicals. The selenium on the other hand forms an integral part of the enzyme glutathione peroxidase, an enzyme which catalyses the chemical decomposition of biologically generated peroxides. Together, vitamins C and E and glutathione peroxidase form part of the body's important protective mechanism against free radical damage. The anti-oxidants, in particular vitamin C, also have the ability to counteract the effects of some of the products of free radical and oxygen activity already present in infant food formulations such as the aforesaid oxidised lipids.

Such anti-oxidants also reduce the risk of free radical damage resulting from excessive homocysteine accumulations in infants.

4

EP 0 595 006 A1

Accordingly, the present invention further proposes the preferred incorporation in the preparations according to the invention of an anti-oxidant preferably one or more of the substances d) vitamin C, e) vitamin E (e.g. as tocopherol acetate) and f) selenium (e.g. as selenium yeast), more particularly in amounts to provide the following daily dosages per infant:

d) 10-100 mg, preferably 20-80 mg, more preferably 40-70 mg, e.g. 50 mg

e) 0,1-20 mg, preferably 0,5-15 mg, more preferably 1-10 mg, e.g. 4 mg

f) 1-80 $\mu$g, preferably 3-20 $\mu$g, more preferably 5-15 $\mu$g.

From the aforegoing the range of suitable ratios in relation e.g. to vitamin B6 may be calculated.

Alternative or additional anti-oxidants for purposes of the present invention include carotenes (preferably $\beta$-carotene), ubiquinone and vitamin A.

Marginal zinc deficiencies are widespread in infants, particularly when bottle-fed. Applicant has surprisingly found that this is mainly due to poor absorption of zinc from cow's milk and proprietary infant foods. This problem can, according to the invention, be overcome by the inclusion of zinc in the preparation in the presence of substrates that will enhance and facilitate zinc absorption in the infant. According to the invention vitamin C and/or citrate are suitable for that purpose, and the incorporation of zinc citrate plus vitamin C is therefore a preferred integer of the invention. Zinc (in the form of zinc citrate) is advantageously employed in daily dosages (calculated as Zn) in the range of 0,1-20 mg, preferably 0,5-10, more preferably 1-8 mg, e.g. 4 mg Zn.

Human milk, but not cow's milk, contains substantial quantities of gamma linolenic acid (GLA), an important precursor of the prostaglandin 1-series in humans. Applicant has found that in some infants, especially premature infants, the enzymes required for producing GLA from its natural precursors are underdeveloped, resulting in further potential complications in addition to those outlined above. Accordingly the invention proposes in addition the incorporation in the preparation of GLA, to provide a daily dosage of 2-20 mg, preferably 6-10 mg.

Finally, because of the frequency of food allergies arising in bottle-fed infants, the invention proposes the incorporation in the preparation of an allergy suppressing substance. It has been found that extracts of finely ground plant material from the herb tea plant Aspalatus contaminatus druce or related species suitable for that purpose and is not only compatible with the above described ingredients of the preparation, but that its effect is in fact enhanced by these. This ingredient is employed in daily dosages of 1-20 mg, preferably 8-12 mg.

The general pharmacological and toxicological properties of the individual ingredients of the preparations in accordance with the invention are well documented in the art and require no further description. The dosage rates in accordance with the invention are below accepted toxicity levels.

The effectiveness of the claimed combination for reducing elevated homocysteine has been tested in adults as described in the abovementioned co-pending application. The results of those tests can be extrapolated to infants. It was shown that the effect of the combination taught by the present invention is greater than the mere additive effect of individual ingredients.

## EXAMPLE

Rapidly dissolving tablets were made as follows:-

| Per tablet | |
|---|---|
| Pyridoxal (as pyridoxal hydrochloride) | 0,10 mg |
| Pyridoxine (as pyridoxine hydrochloride) | 0,05 mg |
| Riboflavin (as riboflavin phosphate sodium) | 0,10 mg |
| Ascorbic Acid | 25,0 mg |
| Vit E (as d-alpha-tocopherol acetate) | 2,0 mg |
| Folic acid | 0,02 mg |
| Cyanocobalamin | 0,00055 mg |
| Zinc (as zinc citrate) | 2,0 mg |
| Selenium (as selenised yeast) | 5,0 microgram |
| Avicel | 45 mg |
| Magnesium, stearate | 0,05 mg |
| Aerosil | 0,05 mg |

5

Tablets were prepared in the usual manner. One tablet is added twice daily to the infant's bottle feed immediately before feeding.

Total daily dose: 2 tablets.

**The pharmacology and toxicology** of all active substances employed in accordance with the present invention is known per se and requires no description. As regards evidence of synergism between these ingredients reference is made to our copending application claiming the priority of South African patent application 92/6990.

**Clinical tests on infant formulations**

A) The following are results of measurements of the vitamin status in serum of diverse infants. In the table the prefix "S" denotes "Serum".

| BABY | AGE (days) | S-PLP nmol/ml | S-PL nmol/ml | S-PL / S-PLP | S-B12 pmol/ml | S FOLATE nmol/ml | S-HC umol/ml |
|---|---|---|---|---|---|---|---|
| MOTHER'S MILK PLUS FORMULA | | | | | | | |
| 1.SR | 23 | 49.8 | 324 | 6.5 | 725 | 45 | 5.1 |
| 2.SM | 14 | 66.1 | 465 | 7.1 | 504 | 31.7 | 3.9 |
| 3.SL | 5 | 38.5 | 48.7 | 1.3 | – | – | 10.8 |
| 4.IS* | 8 | 15.6 | 143.3 | 9.2 | 452 | 26.6 | 6.1 |
| 5.JM* | 20 | 52.3 | 306 | 5.9 | 250 | 45 | 7.3 |
| 6.ME* | 10 | 52.2 | 303 | 5.8 | 478 | 26.9 | 5.7 |
| 7.IK* | 7 | 63 | 167 | 2.7 | 418 | 27.1 | 4.2 |
| 8.KM | 4 | 12.1 | 52.5 | 4.3 | 311 | 45 | 5.1 |
| 9.ML** | 4 | 14.2 | 9.4 | 0.6 | – | – | 6.5 |
| 10.FV | 9 | 129.1 | 309 | 2.4 | 707 | 45 | 7.2 |
| FORMULA only | | | | | | | |
| 11.JB* | 12 | 70 | 464 | 6.6 | 290 | 45 | 8.5 |
| 12.RM | 3 | 32 | 14.4 | 0.4 | 658 | 45 | 6.5 |
| BREAST only | | | | | | | |
| 13.VM | 4 | 44.9 | 17.1 | 0.4 | 225 | 45 | 7.4 |
| HYPERALIMENTATION | | | | | | | |
| 14.EM | 11 | 4.9 | 11.7 | 2.4 | 541 | 28.4 | 4.7 |

\* Premature birth

\*\* Diabetic

B) Blood levels of PL in premature infants receiving feed supplement in accordance with the invention are summarised in the following:

Average Gestational age: 27 weeks

Two groups, (n = 7) each

Both groups were on a normal hospital feeding programme bottle feed for premature infants. The experimental group received additionally two tablets of a rapidly dissolving PL containing tablet according to the invention, (0,1 mgPL/tablet) daily. None received any mothers milk.

**Results** (average values)

**S-PLP** (nmol/*l*

|  |  | Admission | 2 | 5 | 7 | 14 | days |
|---|---|---|---|---|---|---|---|
|  | Control | 24* | 11 | 7 | 7.5 | 8 |  |
|  | Exp. | 27* | 10 | 9 | 6 | 7 |  |

**S-PL** (nmol/*l*)

|  |  | Admission |  | 2 | 5 | 7 | 14 |
|---|---|---|---|---|---|---|---|
|  | Control | 190 |  | 181 | 196 | 191 | 174 |
|  | Exp | 181 |  | 196 | 221 | 229 | 246 |

\*)     Immediately after birth: still from mother, rapid drop during first 3 days.

C) For ethical reasons it was not possible to obtain repeated blood samples from infants during comprehensive clinical trials. Reliance is therefore placed on trials performed on human adults as reported in the copending application claiming the priority of South African patent application 92/6990, which show that for humans of all age groups, in order to lower elevated homocysteine levels in plasma (which are frequently paralled by methionine levels) the following was found:-
PL supplementation alone: 18,8% success
Vitamin B12 supplementation alone:31,3% success
folate supplementation alone:62,5% success
combination of all three supplementation alone:91,7% success after 6 weeks and 100% after 8 weeks.
There was clear evidence of synergism between the three components.

D) **Summary of clinical findings**

1. In healthy infants plasma PL levels are very much higher than in adults. The ratio of PL:PLP in plasma in infants is approximately 10 or more times as high as in adults. This shows that PL in plasma is of fundamental importance to human infants.

2. Low plasma P/PLP ratios can often be correlated with abnormally high plasma-homocysteine levels in infants.

3. In other infants high plasma-homocysteine levels can be correlated with low serum B12 and/or folate levels.

4. PL supplementation in infants, in particular premature infants increases plasma PL and intracellular PLP levels, but not plasma PLP levels (the latter was also found by Raiten, Reynolds, et al (Am J.Clin. Nutr. 1991; 53:78-83). Plasma PLP is unimportant in infants.

5. Premature infants (gestational age 30 weeks or less) are generally unable to effectively convert PN into PL. The ability to so convert improves (to a highly variable extent) both with gestational age and age after birth, although approximately 20% remain poor converters into adulthood. To compensate for this by prior art high dosage PN supplementation is not possible and produces dangerous side effects.

6. The formula for infants has been confirmed, based on the clinical trials C) on adults, duly adapted in the light of the clinical findings as per A) and B).

**Claims**

1. A pharmaceutical or dietary preparation said preparation being either galenically prepared for parenteral use, in particular by infusion, or as a supplement for addition to an infant feed preparation, in the form of paediatric drops, or in another dosage form specifically for paediatric use, said preparation comprising in combination:-

   a) vitamin B 6 as such or in the form of a pharmaceutically acceptable acid salt, at least in part in the form of pyridoxal (PL) or a compound which in vivo readily releases PL without the intervention of oxidase enzyme or oxygen.

   b) folate or a precursor of folate which releases folate in vivo, and

   c) vitamin B12, with or without intrinsic factor, in the following ratios:-

   a) : b) from 1:25 to 10 000 : 1

   b) : c) from 1:1 to 50 000 : 1

2. The use of the substances hereinafter set out for the manufacture of a pharmaceutical or dietary preparation for the treatment or prophylaxis of elevated homocysteine and/or methionine levels in the blood of human infants and pathological disturbances connected therewith, said preparation comprising in combination:-

   a) vitamin B 6 as such or in the form of a pharmaceutically acceptable acid salt, at least in part in the form of pyridoxal (PL) or a compound which in vivo readily releases PL without the intervention of oxidase enzyme or oxygen.

   b) folate or a precursor of folate which releases folate in vivo, and

   c) vitamin B12, with or without intrinsic factor, in the following ratios:-

   a) : b) from 1:25 to 10 000 : 1

   b) : c) from 1:1 to 50 000 : 1

3. An infant bottle feed preparation, said preparation comprising in combination:-

   a) vitamin B 6 as such or in the form of a pharmaceutically acceptable acid salt, at least in part in the form of pyridoxal (PL) or a compound which in vivo readily releases PL without the intervention of oxidase enzyme or oxygen.

   b) folate or a precursor of folate which releases folate in vivo, and

   c) vitamin B12, with or without intrinsic factor, in the following ratios:-

   a) : b) from 1:25 to 10 000 : 1

   b) : c) from 1:1 to 50 000 : 1

4. A method of supplementing a bottle feed composition for infants by introducing into such composition a preparation as claimed in claim 1.

5. The invention as set out in any one of claims 1 to 4, characterised in that said ratios are:

   a) : b) from 2:5 to 150:1

   b) : c) from 2:1 to 4000:1,

   preferably

   a) : b) from 13:1 to 100:1

   b) : c) from 10:1 to 1000:1,

   more preferably

   a) : b) = 15:2

   b) : c) = 400:11

6. The invention as claimed in any one or more of claims 1 to 5, characterised in that the preparation is formulated for oral use.

7. The invention as claimed in any one or more of claims 1 to 6, characterised by the inclusion of PN or PN precursor in the preparation in addition to PL in a ratio of PN:PL in the range 10:1 to 1:10, preferably 4:1 to 1:6, more preferably 2:1 to 1:3, e.g. 1:2.

8. The invention as claimed in claim 7, characterised in that the preparation comprises riboflavin, e.g. in an amount of from 0,5 to 5 times, e.g. twice the amount of PN.

9. The invention as claimed in any one or more of claims 1 to 8, characterised in that the preparation comprises an anti-oxidant preferably one or more of the substances d) vitamin C, e) vitamin E (e.g. as tocopherol acetate) and f) selenium (e.g. as selenium yeast), more particularly in amounts to provide the following daily dosages:
  d) 10-100 mg, preferably 20-80 mg, more preferably 40-70 mg, e.g. 50 mg
  e) 0,1-20 mg, preferably 0,5-15 mg, more preferably 1-10 mg, e.g. 4 mg
  f) 1-80 μg, preferably 3-20 μg, more preferably 5-15 μg.

10. The invention as claimed in any one or more of claims 1 to 9, characterised in that the preparation comprises gamma linolenic acid (LA) to provide a daily dosage of 2-20 mg, preferably 6-10 mg.

11. The invention as claimed in any one or more of claims 1 to 10, characterised in that the preparation also contains zinc, preferably proportioned to provide daily dosages in the range of 0,1-20 mg, preferably 0,5-10, more preferably 1-8 mg, e.g. 4 mg Zn.

12. The invention as claimed in any one or more of claims 1 to 11, characterised in that the preparation also contains an allergy suppressing substance, e.g. derived from the herb tea plant Aspalatus contaminatus Druce or related species suitable for that purpose.

13. The invention as claimed in anyone or more of claims 1 and 3 to 12 for the treatment or prophylaxis of elevated homocysteine and/or methionine levels in the blood of human infants and pathological disturbances connected therewith.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,X, Y | EP-A-0 379 936 (VESTA MEDICINES (PROPRIETARY) LIMITED) * the whole document, especially examples 14 and 15 * | 1-13 | A61K31/68 //(A61K31/68, 31:505,31:44) |
| D,P, X | GB-A-2 254 556 (FISONS PLC) * page 1, line 1-7 * * page 4, line 8-23 * * page 6, line 6-9; claims 1,4,12,13,34,42,44-46 * | 1,5-13 | |
| D,X | GB-A-1 201 014 (E. MERCK AG) * page 2, line 27-38; example 6 * | 1,5-13 | |
| X | WO-A-91 11117 (BOARD OF REGENTS, THE UNIVERSITY OF TEXAS SYSTEM) * abstract * * page 23, line 29 - page 24, line 2; claims * | 1,5-13 | |
| X | DIALOG INFORMATION SERVICES, INC., FILE 446: IMSWORLD NEW PRODUCT LAUNCHES, AN=01238461 * Hepabionta * | 1,5-13 | **TECHNICAL FIELDS SEARCHED** (Int.Cl.5) A61K |
| X | DIALOG INFORMATION SERVICES, INC., FILE 446: IMSWORLD NEW PRODUCT LAUNCHES, AN=01235212 * Anemo Funk * | 1,5-13 | |
| X | DIALOG INFORMATION SERVICES, INC., FILE 446: IMSWORLD NEW PRODUCT LAUNCHES, AN=01240111 * Normovite Antianem * | 1,5-13 | |

-/--

The present search report has been drawn up for all claims

| Place of search | Date of completion of the search | Examiner |
|---|---|---|
| THE HAGUE | 31 January 1994 | Orviz Diaz, P |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | DIALOG INFORMATION SERVICES, INC., FILE 446: IMSWORLD NEW PRODUCT LAUNCHES, AN=01148023 * Melicedin SRS * | 1,5-13 | |
| P,X, L | AM. J. CLIN. NUTR vol. 57, no. 1 , January 1993 pages 47 - 53 J.B. UBBINK 'Vitamin B-12, vitamin B-6, and folate nutritional status in men with hyperhomocysteinemia' * the whole article, especially page 49, left column, lines 1-5* (The vitamin supplement ROYL-CYNOR is mentioned) | 1-13 | |
| P,X | CLIN. INVEST. vol. 71, no. 12 , 1993 pages 993 - 998 J.B. UBBINK 'Hyperhomocysteinemia and the response to vitamin supplementation.' * the whole document * | 1-13 | TECHNICAL FIELDS SEARCHED (Int.Cl.5) |
| X | ATHEROSCLEROSIS vol. 75, no. 1 , 1989 pages 1 - 6 A.J. OLSZEWSKI 'Reduction of plasma lipid and homocysteine levels by pyridoxine, folate, cobalamin, choline, riboflavin, and troxerutin in atherosclerosis.' * the whole document * | 1-13 | |

-/--

The present search report has been drawn up for all claims

| Place of search | Date of completion of the search | Examiner |
|---|---|---|
| THE HAGUE | 31 January 1994 | Orviz Diaz, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| L | ATHEROSCLEROSIS<br>vol. 88, no. 1 , 1991<br>pages 97 - 98<br>A.J. OLSZEWSKI 'Homocysteine content of plasma in ischemic heart disease, the reducing effect of pyridoxine, folate, cobalamin, choline, riboflavin, and troxerutin. Correction of a calculation error.'<br>* the whole document *<br>(Correction of the results described in the previous reference) | 1-13 | |
| D,Y | SCAND. J. CLIN. LAB. INVEST.<br>vol. 48, no. 3 , 1988<br>pages 215 - 221<br>L.E. BRATTSTRÖM 'Folic acid-an inocuous means to reduce plasma homocysteine.'<br>* the whole document * | 1-13 | |
| Y | ATHEROSCLEROSIS<br>vol. 81, no. 1 , 1990<br>pages 51 - 60<br>L. BRATTSTRÖM 'Impaired homocysteine metabolism in early-onset cerebral and peripheral occlusive arterial disease.'<br>* the whole document * | 1-13 | TECHNICAL FIELDS SEARCHED (Int.Cl.5) |
| Y | TRENDS PHARMACOL. SCI.<br>vol. 11, no. 10 , 1990<br>pages 411 - 416<br>H. REFSUM 'Clinical significance of pharmacological modulation of homocysteine metabolism.'<br>* the whole document *<br>-/-- | 1-13 | |

The present search report has been drawn up for all claims

| Place of search | Date of completion of the search | Examiner |
|---|---|---|
| THE HAGUE | 31 January 1994 | Orviz Diaz, P |

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 270 026 (VESTA MEDICINES (PROPRIETARY) LIMITED) * claims * | 1-13 | |
| Y | FRISOLAC, PRODUCT INFORMATION * Analysis * | 1-13 | |
| Y | ROTE LISTE. BUNDESVERBAND DER PHARMAZEUTISCHEN INDUSTRIE E.V., EDITIO CANTOR, AULENDORF, 1992. * preparations containing vitamin B6, vitamin B12, and folic acid * | 1-13 | |

TECHNICAL FIELDS
SEARCHED (Int.Cl.5)

The present search report has been drawn up for all claims

| Place of search | Date of completion of the search | Examiner |
|---|---|---|
| THE HAGUE | 31 January 1994 | Orviz Diaz, P |

EPO FORM 1503 03.82 (P04C01)